# EUROPÄISCHE PATENTSCHRIFT

(11) **EP 1 593 350 B1**
(45) Veröffentlichungstag und Bekanntmachung des Hinweises auf die Patenterteilung: **13.06.2007**
(21) Anmeldenummer: 04010669.2
(22) Anmeldetag: 05.05.2004
(51) Int. Cl.: A61B 19/00

(54) **Marknagel-Tracking**
Tracking of intramedullary pin
Localisation d'un clou centromedullaire

(43) Veröffentlichungstag der Anmeldung: 09.11.2005
(73) Patentinhaber: BrainLAB AG, 85551 Kirchheim/Heimstetten (DE)
(72) Erfinder: Tuma, Gregor, 81675 München (DE); Wende, Christian, 82178 Puchheim (DE)
(74) Vertreter: Schwabe - Sandmair - Marx

(56) Entgegenhaltungen:
- WO-A-03/068090
- WO-A-20/04034914
- US-A- 5 540 691

## Beschreibung

Die Erfindung betrifft das Gebiet des Marknagel-Tracking. Insbesondere betrifft sie eine Trackingvorrichtung für einen Marknagel, dessen räumliche Lage bei der bildschirmunterstützten Chirurgie bestimmt bzw. verfolgt wird, mit einer Referenzeinrichtung am proximalen Ende des Marknagels und mit einem medizinischen Navigationssystem, das die Referenzeinrichtung ortet sowie die Ausrichtung des Marknagels vom proximalen Ende her bestimmt.

Die Verwendung von Marknägeln ist eine bekannte und etablierte Behandlungsform für Brüche langer Knochen. Am proximalen und distalen Ende des Marknagels (Implantats) sind Arretierungslöcher vorgesehen, in die Schrauben eingesetzt werden können, um den Marknagel und die Knochenfragmente gegeneinander zu fixieren.

Ein Hauptproblem besteht dabei darin, dass sich der implantierte Marknagel beim Einbringen in den Knochen verformt bzw. deformiert, und zwar durch die individuelle Form des langen Knochens. Wegen dieser Deformation ist dann grundsätzlich die Lage und Ausrichtung des distalen Endes des Marknagels unbekannt, und damit natürlich auch die Lage und Ausrichtung der distalen Arretierungsbohrung. Um die Fixierungsschrauben einsetzen zu können, muss die Lage der distalen Arretierungsbohrung aber genau bestimmt werden, und dies geschieht herkömmlicherweise mit Hilfe von Röntgenbildern. Meist verwenden die Chirurgen C-Bogen-Fluoroskopiegeräte um die Nagel- bzw. Bohrungslage zu bestimmen und die Schraube am distalen Ende einsetzen zu können.

Nachteilig dabei ist die Strahlenbelastung für den Patienten und das Behandlungsteam. Weiterhin nachteilig ist für den Operateur bei der Anwendung von C-Bögen das Fehlen der dritten Dimension. Anhand von intraoperativ erstellten Röntgenprojektionen auch aus mehreren Winkeln fehlt in der Regel noch die ausreichende Orientierung im Raum, um sicher in allen drei Dimensionen und allen Freiheitsgraden die Schraubeneinbringung kontrollieren zu können.

Um diese Probleme zu lösen hat man einerseits versucht, ein optisches Zielsystem zu verwenden, wie es beispielsweise aus der US 5,417,688 bekannt ist. Dabei wird eine Lichtquelle an das distale Ende des Marknagels angebracht und verwendet, um die Ausrichtung des distalen Schrauben-Arretierungsloches zu bestimmen. Der Chirurg soll dabei die auf die Oberfläche des Körpers des Patienten übertragene Lichtstrahlung benutzen, um seinen Bohrer danach auszurichten. Nachteilig ist dieses Verfahren, weil es ungenau ist und die Lichtquelle von außen nur schlecht sichtbar sein dürfte.

Andererseits ist vorgeschlagen worden, magnetische Zielsysteme zu verwenden, wie beispielsweise in der US 6,162,228, der US 5,411,503 und der US 5,584,838. Dabei wird im distalen Ende des Marknagels eine spezielle Konfiguration von Magneten angebracht oder ein vorbestimmtes magnetisches Feld erzeugt, um die Ausrichtung des distalen Arretierungsloches zu bestimmen. Der Chirurg verwendet ein Navigationswerkzeug, das mit einem magnetischen Sensorsystem versehen ist, um den Marknagel zu arretieren. Nachteilig ist bei diesen magnetischen Systemen immer, dass sie aufwendig und relativ störungsanfällig sind. Letzteres gilt besonders dann, wenn Stahlnägel verwendet werden.

Aus der WO 03/068090 A1 ist ein getrackter Manknagel bekannt, der eine Referenz aufweist, die in einem vorbestimmten körperlichen Verhältnis zum länglichen Nagelkörper roteht. Dieses Dokument offenbart die im einführenden Teil des Anspruchs 1 definierten Merkmale.

Ein weiterer herkömmlicher Ansatz basiert darauf, existierende Navigationsverfahren zu verwenden, die ein Tracking des Nagels anbieten, und einen C-Bogen in die Navigation zu integrieren. Bei diesen Systemen ergab die Erfahrung, dass ein alleiniges Tracking des Nagels ohne die Berücksichtung der Deformation in der Regel trotz des Einsatzes von Fluoroskopie in Kombination mit Navigation zu Fehlbohrungen führt. Ein weiterer Nachteil dieser bekannten Verfahren liegt darin, dass sie nach wie vor auf dem Einsatz von Strahlung (C-Bogen) zur Bildgebung basieren und die Lage der Durchgangsbohrung manuell und ungenau (durch z.B. Fehlinterpretation der dargestellten Projektion) im Bild festgelegt wird. Um in den C-Bogen-Bildern navigieren zu können, müssen sowohl die Bilder in kalibierter Form vorliegen (entzerrt und unter Bekanntheit der räumlichen Lage der Projektion bei der Aufnahme), als auch die Knochenstruktur, in der sich der Marknagel befindet, mit einer Referenzanordnung versehen sein (zusätzliche Invasivität).

Es ist deshalb die Aufgabe der vorliegenden Erfindung, ein praxisfähiges Marknagel-Tracking bereitzustellen. Insbesondere soll bei der Arretierung des Marknagels genau gearbeitet werden können, möglichst ohne Störanfälligkeiten. Ferner sollen Strahlenbelastungen vermieden werden.

Diese Aufgabe wird erfindungsgemäß bei einer Trackingvorrichtung für einen Marknagel dadurch gelöst, dass eine Deformations-Detektionseinrichtung bereitgestellt wird, die sich entlang des Marknagels erstreckt, die Deformation des Marknagels erfasst und dem Navigationssystem mitteilt. Die Unteransprüche definieren bevorzugte Ausführungsformen der Erfindung.

Der Vorteil, der mit der vorliegenden Erfindung erzielt werden kann, liegt insbesondere in der genauen und patientenfreundlichen Navigation, die ermöglicht wird. Die Information über die Lage des distalen Endes des Marknagels wird praktisch aus zwei Informationen zusammengesetzt, nämlich aus der absoluten Position des proximalen Endes (durch die Referenzeinrichtung) und aus der Information über die Deformation, also einer relativen Information gegenüber dem undeformierten Nagel. Damit wird von dem Prinzip abgewichen, das bisher sowohl bei optischen als auch bei magnetischen Zielfindungsverfahren verwendet wurde, nämlich von dem Prinzip der direkten und absoluten Bestimmung der Lage und Ausrichtung des distalen Marknagelendes bzw. der Bohrung. Störungen und Ungenauigkeiten, die bei solchen direkten Bestimmungen aufgrund des außen um den Marknagel liegenden Patientengewebes immer auftreten, werden überwunden. Strahlenbelastungen werden vermieden, und das Erreichen der Ziel-Trajektorie der Durchgangsbohrung im Marknagel kann mit der erfindungsgemäßen Lösung vollständig ohne Einsatz von Strahlung in allen Freiheitsgraden (drei Dimensionen) kontrolliert werden. Die Anordnung von Referenzklemmen ist im erfindungsgemäßen Konzept nicht zwingend notwendig; dies macht das Verfahren schneller, leichter und weniger belastend für Arzt und Patient (weniger invasiv).

Gemäß einer bevorzugten Ausführungsform erfasst die Deformation-Detektionseinrichtung Biegungen und/oder Torsionen des Marknagels. Insbesondere kann sich die Deformations-Detektionseinrichtung im Wesentlichen vom proximalen Ende bis im Wesentlichen zum distalen Ende des Marknagels erstrecken, bevorzugt von der proximalen bis zur distalen Festlegungseinrichtung, insbesondere Arretierungsbohrung des Nagels.

Vorteilhafterweise befindet sich die Deformation-Detektionseinrichtung im Inneren des Marknagels, und sie kann eine Lichtleiterstruktur, insbesondere ein Glasfaser-Lichtleiterstruktur sein, die Deformationen aufgrund von Licht-Laufzeitunterschieden erfasst. Diese Lichtleiterstruktur kann einerseits eine Struktur mit hintereinander angeordneten Lichtleiterschleifen aufweisen, andererseits oder zusätzlich auch mindestens zwei in einem Winkel zueinander ausgerichtete, insbesondere senkrecht zueinander stehende Schleifenstrukturen umfassen, um Torsionen gut bestimmbar zu machen.

Bei dem Trackingverfahren zur Bestimmung bzw. Verfolgung der räumlichen Lage eines Marknagels bei der bitdunterstützten Chirurgie wird eine Referenzeinrichtung am proximalen Ende des Marknagels angeordnet und mit einem medizinischen Navigationssystem geortet, und die Ausrichtung des Marknagels vom proximalen Ende her wird bestimmt. Dabei werden dann mittels einer Deformation-Detektionseinrichtung, die sich entlang des Marknagels erstreckt, Deformationen des Marknagels erfasst und dem Navigationssystem mitgeteilt. Mittels dieser Informationen kann die Lage des Marknagels nach der Deformation dann im Navigationssystem dargestellt werden und mit Hilfe dieser Informationen können wiederum erfasste Biegungen und/oder Torsionen des Marknagels bei der Berechnung der Ausrichtung einer Arretierungsbohrung am distalen Ende verwendet werden, um ein genaues Einsetzen einer Arretierungsschraube zu ermöglichen.

Anders ausgedrückt werden durch die vorliegende Erfindung mehrere unterschiedliche Technologien kombiniert, nämlich
1. die virtuelle Navigation einer nichtelastischen Vorrichtung mit Hilfe der bildgestützten Chirurgie (optisches Trackingsystem),
2. die virtuelle Deformations-Detektion, bei der Deformationen erfasst, errechnet und visualisiert werden,
3. die Verwendung von Marknägeln, und
4. die Verwendung von Zielsystemen zum Arretieren von Marknägeln.

Bei der virtuellen Navigation (1.) kann die Nagelgeometrie (und damit der Lage der Fixierschrauben-Durchgangstrajektorie) in einer Datenbank hinterlegt sein, um genügend Informationen bereitzustellen, so dass eine räumliche Lageänderung der Durchgangstrajektorie durch die Marknageldeformation vollständig beschrieben werden kann. Alternativ gibt es die Möglichkeit ohne Datenbank zu arbeiten. Die Trajektorie kann dann vor Einsetzen des Nagels (undeformiert) festgelegt ("kalibiert") und dem Navigationsystem bekannt gemacht werden, das heißt die Bohrungs-Durchgangstrajektorie wird auf die Referenzstruktur registriert. Dabei kann zum Beispiel ein Pointer oder ein Bohrer oder eine Schraube mit einer eigenen Referenzanordnung durch die Bohrung des Marknagels geführt und der Marknagel mit seiner Referenzanordnung in das Kamarasystem des Navigationssystems gehalten werden, um der Navigationssoftware die Trajektorienorientierung des undeformierten Nagels mitzuteilen.

Die letztgenannten Zielsysteme (4.) können die Informationen verarbeiten, welche dem Navigationssystem nach der erfindungsgemäßen Lageerfassung zur Verfügung stehen.

Die Erfindung wird im Weiteren anhand eines Ausführungsbeispiels näher erläutert. In den Zeichnungen, auf die hierbei Bezug genommen wird, zeigen:
- Figur 1: eine schematische Darstellung eines Marknagels mit einem Setzhilfen-Aufsatz;
- Figur 2: den Marknagel im deformierten Zustand, eingesetzt in einen Knochen, sowie das erfindungsgemäße Trackingsystem;
- Figur 3 und Figur 4: Lichtleiterstruktur-Anordnungen in einem Marknagel.

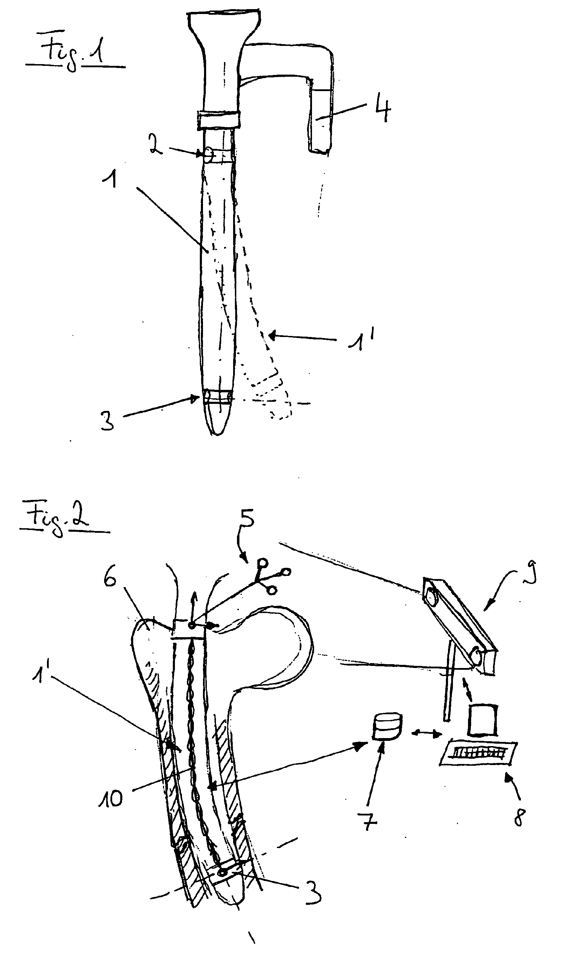

Die Figur 1 zeigt den Marknagel 1 in seiner undeformierten Gestalt sowie gestrichelt einen deformierten Marknagel 1'. Der Marknagel 1 weist an seinem proximalen Ende die Arretierungsbohrung 2 und seinem distalen Ende die Arretierungsbohrung 3 auf. Ferner ist er an einem Setzhilfen-Aufsatz 4 angebracht, mit Hilfe dessen er in den Knochen eingeführt werden kann.

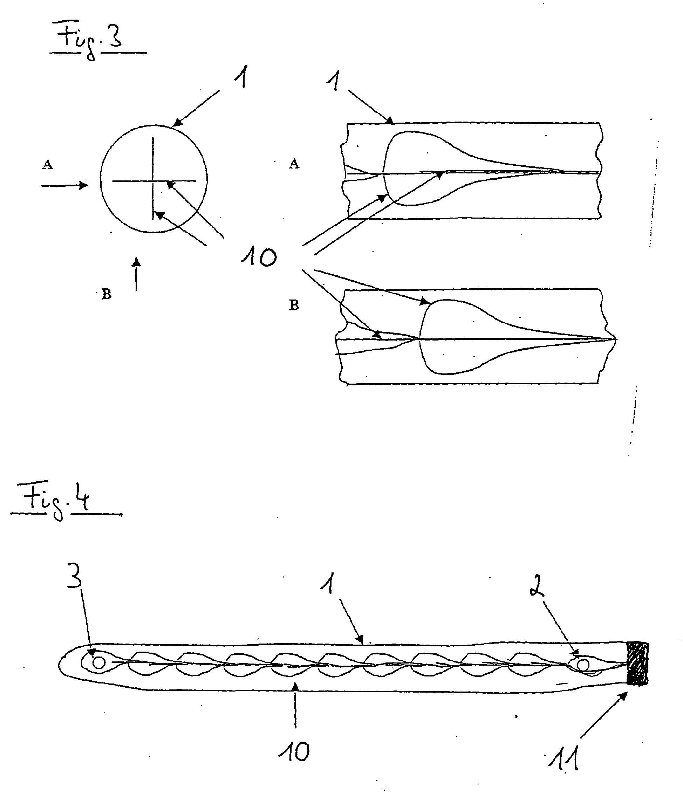

Der Nagel 1', wie er in einem Knochen eingesetzt und dabei deformiert worden ist, ist in Figur 2 ersichtlich. Der Knochen 6 weist eine Biegung auf, welcher der Marknagel beim Einsetzen folgt, was dazu führt, dass die Arretierungsbohrung 3 am distalen Ende schräg zu liegen kommt. Um die Lage und Ausrichtung der Bohrung 3 zu bestimmen und damit eine Arretierungsschraube richtig einsetzen zu können, bedient man sich nun des erfindungsgemäßen Tracking-Systems. Dieses umfasst am proximalen Ende, wo der Nagel 1' noch aus dem Knochen herausragt, eine Referenzstruktur 5, beispielsweise eine dreiarmige Referenzanordnung mit passiven, also rückstrahlenden Markerkugeln. Diese Referenzanordnung kann von einem Navigationssystem geortet werden, das hier nur schematisch dargestellt ist und eine Kameraanordnung 9 sowie einen Computer 8 mit Bildschirmausgabe umfasst. Der Computer 8 umfasst auch eine Datenbank 7, in der die Informationen über die ursprüngliche Gestalt des Marknagels hinterlegt sind. Mit Hilfe des Navigationssystems 7, 8, 9, das die Referenzanordnung 5 orten kann, ist demnach die Position des Marknagels am proximalen Ende bekannt sowie die Richtung, in die sich der Marknagel erstrecken würde, wenn er nicht deformiert wäre.

Um nun die Deformation des Marknagels, also beispielsweise die Biegung und Torsion erfassen zu können und diese Werte denjenigen Werten zu überlagern, die vom Referenzstern 5 her stammen, um damit die Lage und Ausrichtung der Bohrung 3 zu wissen, ist innerhalb des Marknagels 1' eine Deformation-Detektionseinrichtung 10 angeordnet. Diese Einrichtung weist verschiedene Glasfaserstreifen bzw. - schleifen auf, deren Anordnung aus den Figuren 3 und 4 hervorgeht. Die Figur 3 zeigt links einen Querschnitt durch den Marknagel 1 aus dem zu erkennen ist, dass hier zwei Lichtleiterstrukturen senkrecht zueinander angeordnet sind. Die Ansichten A und B, die links am Querschnitt angezeigt werden, sind rechts vom Querschnitt nochmals gezeigt; man erkennt ebenfalls die schleifenförmigen Lichtleiter-Glasfaserstreifen 10. Die senkrecht zueinander angeordneten Schleifen 10 sind auch axial zueinander versetzt. Die Kenntnis der Lage der Trajektiorie relativ zum Referenzstern 5 im undeformierten Zustand ist in der Datenbank 7 hinterlegt (bzw. durch Kalibrieren vorher dem System bekannt gemacht). Durch Überlagerung der Deformationen und der Trackinginformation erhält man die veränderte Lage dieser Trajektorie.

Die Figur 4 zeigt noch, dass die Schleifen der Glasfaseranordnung sich vom proximalen Arretierungsloch 2 zum distalen Arretierungsloch 3 im Marknagel 1 erstrecken, damit der gesamte interessierende Bereich seiner Deformation nach erfasst werden kann. Am rechten Ende des Marknagels 1 in Figur 4 ist noch schematisch eine Schnittstelle 11 zur Übertragung der Deformationsdaten zum Navigationssystem gezeigt. Die Übertragung kann per Kabel oder auch kabellos erfolgen.

Bei einer Deformation erfährt jede Glasfaser eine individuelle Laufzeitänderung für ihren Lichtstrahl, und auf der Basis der resultierenden Verzögerung kann die Deformation errechnet werden. Der integrierte Glasfasersensor wird also eine Deformations-Rückmeldung für den Marknagel in Echtzeit zum Navigationssystem senden können. Das Navigationssystem stellt den Marknagel gegenüber einem bereits registrierten Patientenkörperteil (Knochen) wiederum in Echtzeit so dar, wie er tatsächlich liegt, und mit Hilfe dieser Visualisierung kann dann die Einbringung der distalen Arretierungsschraube genau vorgenommen werden.

## Patentansprüche

1. Trackingvorrichtung für einen Marknagel (1), dessen räumliche Lage bei der bildunterstützten Chirurgie bestimmt bzw. verfolgt wird, mit einer am proximalen Ende des Marknagels (1) festlegbaren Referenzeinrichtung (5) und mit einem medizinischen Navigationssystem (7, 8, 9), das die Referenzeinrichtung (5) ortet, **gekennzeichnet durch** eine Deformations-Detektionseinrichtung (10), die sich entlang des Marknagels (1) erstreckt, Deformationen des Marknagels (1) erfasst und dem Navigationssystem (7, 8, 9) mitteilt und die Ausrichtung des Marknagels (1) vom proximalen Ende her bestimmt.

2. Trackingvorrichtung nach Anspruch 1, **dadurch gekennzeichnet, dass** die Deformations-Detektionseinrichtung (10) Biegungen und/oder Torsionen des Marknagels (1) erfasst.

3. Trackingvorrichtung nach Anspruch 1 oder Anspruch 2, **dadurch gekennzeichnet, dass** die Deformations-Detektionseinrichtung (10) sich im wesentlichen vom proximalen Ende bis im wesentlichen zum distalen Ende des Marknagels (1) erstreckt.

4. Trackingvorrichtung nach Anspruch 3, **dadurch gekennzeichnet, dass** die Deformations-Detektionseinrichtung (10) sich zumindest von der proximalen bis zu der distalen Festlegungseinrichtung, insbesondere Arretierungsbohrung (2, 3), des Nagels (1) erstreckt.

5. Trackingvorrichtung nach einem der Ansprüche 1 bis 4, **dadurch gekennzeichnet, dass** die Deformations-Detektionseinrichtung (10) sich im Inneren des Marknagels (1) erstreckt.

6. Trackingvorrichtung nach einem der Ansprüche 1 bis 5, **dadurch gekennzeichnet, dass** die Deformations-Detektionseinrichtung eine Lichtleiterstruktur, insbesondere eine Glasfaser-Lichtleiterstruktur, ist, die Deformationen aufgrund von Licht-Laufzeitunterschieden erfasst.

7. Trackingvorrichtung nach Anspruch 6, **dadurch gekennzeichnet, dass** die Lichtleiterstruktur (10) eine Struktur mit hintereinander angeordneten Lichtleiterschleifen aufweist.

8. Trackingvorrichtung nach Anspruch 6 oder 7, **dadurch gekennzeichnet dass** die Lichtleiterstruktur (10) mindestens zwei in einem Winkel zueinander ausgerichtete, insbesondere senkrecht zueinander stehende, Schleifenstrukturen aufweist.

## Claims

1. A tracking device for an intramedullary pin (1) whose spatial position is determined and/or tracked in image-guided surgery, comprising a reference means (5) which can be determined at the proximal end of the intramedullary pin (1), and comprising a medical navigation system (7, 8, 9) which locates the reference means (5), **characterised by** a deformation detection means (10) which extends along the intramedullary pin (1), detects deformations of the intramedullary pin (1) and communicates them to the navigation system (7, 8, 9), and determines the orientation of the intramedullary pin (1) from the proximal end.

2. The tracking device according to claim 1, **characterised in that** the deformation detection means (10) detects bending and/or torsion in the intramedullary pin (1).

3. The tracking device according to claim 1 or claim 2, **characterised in that** the deformation detection means (10) extends at least substantially from the proximal end to substantially the distal end of the intramedullary pin (1).

4. The tracking device according to claim 3, **characterised in that** the deformation detection means (10) extends at least from the proximal to the distal determining means, in particular the fixing bore (2, 3) of the pin (1).

5. The tracking device according to any one of claims 1 to 4, **characterised in that** the deformation detection means (10) extends in the interior of the intramedullary pin (1).

6. The tracking device according to any one of claims 1 to 5, **characterised in that** the deformation detection means is an optical fibre structure, in particular a glass optical fibre structure, which detects deformations on the basis of differences in light transit times.

7. The tracking device according to claim 6, **characterised in that** the optical fibre structure (10) comprises a structure with sequentially arranged optical fibre loops.

8. The tracking device according to claim 6 or 7, **characterised in that** the optical fibre structure (10) comprises at least two loop structures orientated at an angle with respect to each other, in particular perpendicular to each other.

## Revendications

1. Dispositif de localisation d'un clou intramédullaire dont la position dans l'espace est déterminée ou suivie lors d'une opération chirurgicale assistée par imagerie, avec un dispositif de repérage (5) à fixer à l'extrémité proximale du clou intramédullaire (1) et avec un système de navigation médical (7, 8, 9) qui localise le dispositif de repérage (5), **caractérisé par** un dispositif de détection de déformation (10) qui s'étend le long du clou intramédullaire (1), détecte des déformations du clou intramédullaire (1) et les transmet au système de navigation (7, 8, 9), et détermine l'orientation du clou intramédullaire (1) depuis l'extrémité proximale.

2. Dispositif de localisation selon la revendication 1, **caractérisé en ce que** le dispositif de détection de déformation (10) détecte des flexions et/ou des torsions du clou intramédullaire (1).

3. Dispositif de localisation selon la revendication 1 ou la revendication 2, **caractérisé en ce que** le dispositif de détection de déformation (10) s'étend sensiblement depuis l'extrémité proximale jusqu'à sensiblement l'extrémité distale du clou intramédullaire (1).

4. Dispositif de localisation selon la revendication 3, **caractérisé en ce que** le dispositif de détection de déformation (10) s'étend au moins depuis le dispositif de fixation proximal jusqu'au dispositif de fixation distal, en particulier le trou de blocage (2, 3) du clou (1).

5. Dispositif de localisation selon l'une des revendications 1 à 4, **caractérisé en ce que** le dispositif de détection de déformation (10) s'étend à l'intérieur du clou intramédullaire (1).

6. Dispositif de localisation selon l'une des revendications 1 à 5, **caractérisé en ce que** le dispositif de détection de déformation est une structure de guide optique, en particulier une structure de guide optique à fibres de verre qui détecte les déformations sur la base de différences de temps de propagation de lumière.

7. Dispositif de localisation selon la revendication 6, **caractérisé en ce que** la structure de guide optique (10) comporte une structure avec des boucles de guide optique disposées les unes derrière les autres.

8. Dispositif de localisation selon la revendication 6 ou 7, **caractérisé en ce que** la structure de guide optique (10) comporte au moins deux structures à boucles orientées suivant un angle l'une par rapport à l'autre, en particulier perpendiculaires l'une à l'autre.
